# EUROPEAN PATENT SPECIFICATION

(11) **EP 2 222 274 B1**
(45) Date of publication and mention of the grant of the patent: **25.01.2012**
(21) Application number: 08856085.9
(22) Date of filing: 01.12.2008
(51) Int. Cl.: A61K 9/00

(54) **METHODS AND COMPOSITONS FOR ENHANCING THE VIABILITY OF MICRONEEDLE PORES**
VERFAHREN UND ZUSAMMENSETZUNGEN ZUR VERSTÄRKUNG DER LEBENSFÄHIGKEIT VON MIKRONADELPOREN
PROCÉDÉS ET COMPOSITIONS PERMETTANT D'ACCROÎTRE LA DURABILITÉ DE PORES CRÉÉS PAR DES MICRO-AIGUILLES

(30) Priority: 29.11.2007 US 990972 P
(43) Date of publication of application: 01.09.2010
(73) Proprietor: AllTranz Inc., Lexington, KY 40506-0286 (US)
(72) Inventor: STINCHCOMB, Audra, Lynn, Lexington KY 40514 (US); BANKS, Stan, Lee, Frankfort KY 40601-0006 (US)
(74) Representative: Grund, Martin
(86) International application number: PCT/US2008/085195
(87) International publication number: WO 2009/073630

(56) References cited:
- WO-A1-02/080912
- WO-A1-2005/097120
- WO-A1-2006/054315
- WO-A2-03/002094
- WO-A2-2008/013822
- US-A1- 2006 084 942
- US-A1- 2008 051 373

## Description

### FIELD

Described herein is the use of cyclooxygenase ("COX") inhibitors in conjunction with microneedle-treated skin for the transdermal delivery of one or more active pharmaceutical agents to a mammal.

### BACKGROUND

Enhancing the transdermal delivery of an active pharmaceutical agent by use of microneedle treatment has become an important area of research in the field of transdermal drug delivery. Microneedles are generally considered to be structures that are between about 20 µm and about 1000 µm in length capable of puncturing the outermost layer of the epidermis (stratum corneum) to create large-scale openings (relative to the size of the active pharmaceutical agent to be delivered there through) or pores through which one or more active pharmaceutical ingredients can be delivered. The depth of microneedle penetration is sufficient to enhance transdermal drug delivery but not sufficient to stimulate nerve endings. Therefore, the use of microneedles is pain-free. This aspect, as well as their economical and easy use, makes a system incorporating microneedle technology an attractive alternative for transdermal drug delivery.

The active pharmaceutical agents to be delivered in conjunction with microneedle technology range from large oligonucleotides to insulin and highly water soluble compounds. Compared to other methods of physically altering the cutaneous structure to aid in improving transdermal transport, microneedle delivery is a relatively simple technique. Microneedles are all micromachined to increase permeability and decrease skin sensation and come in various forms, such as biodegradable polymers, silicon and stainless steel. Various researchers have studied the effects of microneedle-treated skin on increased permeation of mostly water soluble compounds through microneedle-created aqueous pores. It has been shown that the use of microneedles can enhance the permeation of many compounds including non-viral gene therapy vectors, desmopressin, insulin, and naltrexone. Further, the application of microneedles has been shown to be pain free in comparison to a 26-gauge hypodermic needle.

The effectiveness of microneedles is dependent on the duration of time that the microneedle-created pores in the stratum corneum remain sufficiently open and "un-healed." It is during this time that the enhanced delivery of the active pharmaceutical agent can continue. Recently there have been many determinations in pore lifetime and viability via a number of experiments involving transepidermal water loss, microscopic visualization and pharmacokinetic analysis. Transepidermal water loss ("TEWL") measures the rate at which water escapes from the skin. TEWL values are commonly measured in damaged skin to determine water loss over time as a function of skin repair. By using an evaporimeter, an instrument that measures water loss, damage or changes in skin morphology can be determined by an increase in rate of water loss compared to "normal" skin. It has now been shown that TEWL readings are a valid measurement to observe the status of the permeability barrier.

Occlusive coverings, such as patches, can be used to maintain microneedle-created pores. When a microneedle array was placed on the skin and removed without having been treated with an occlusive patch, the skin healed rapidly and TEWL readings returned to baseline levels within 30 minutes. In contrast, it has been demonstrated that under an occlusive environment, microneedle-created pores remained open for at least 48-72 hours. Likewise, microscopic visualization after staining has revealed that pores were present up to 72 hours. In hairless guinea pigs treated with 6-β-naltrexol hydrochloride, significant enhancement in microneedle pore viability was observed for 48 hours after microneedle exposure and occlusion, compared to untreated skin. It has also been shown that therapeutic levels of naltrexone (2.5 ± 1.1 ng/mL) were achieved when a 16% naltrexone hydrochloride gel was administered to 6 healthy human volunteers after microneedle pretreatment. Further, it has been observed that when used in conjunction with microneedles, steady state naltrexone concentrations were achieved within two hours and remained for 48 hours. These results indicate that microneedle application to skin provides an alternative delivery route to oral, injectables and traditional passive transdermal delivery.

Even with the use of occlusive techniques (*e.g*., a patch), in conjunction with the microneedle-generated pores, it is, nevertheless, desirable to further extend the lifetime of microneedle-created pores. Such an increase in the duration of the pore opening can correspond to an increase in the interval between which doses are administered. Said differently, by increasing the duration of the pore opening, it is possible to reduce the frequency with which an active must be administered. Reductions in the dosage frequency have a positive impact on patient acceptance and compliance. Thus, it would be desirable to further enhance the viability of the microneedle-created pores in order to increase the rate, duration and extent of transdermal delivery of an active pharmaceutical agent.

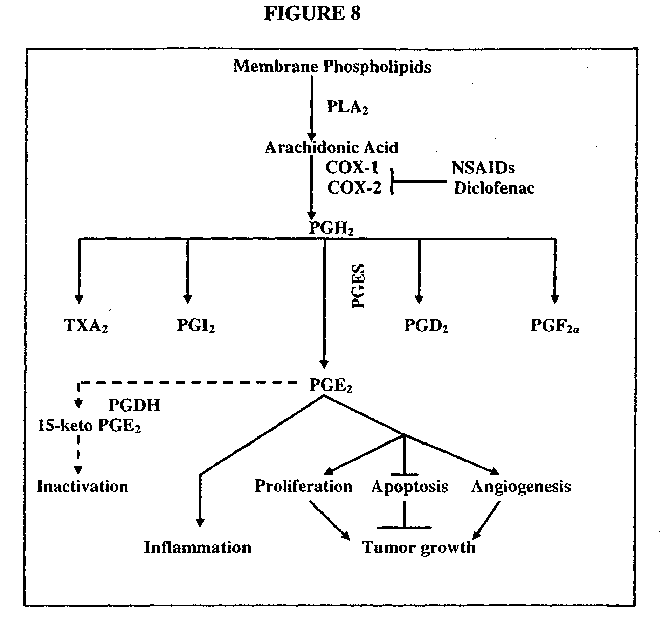

The COX enzymes play a key role in biosynthesis of prostaglandins from arachidonic acid, specifically PGE2. The role of the COX enzymes in the cascade of cellular and genetic events associated with the biosynthesis of prostaglandins from arachidonic acid is illustrated in Figure 8. Prostaglandins, including PGE2, play an essential role in modulating and regulating the inflammatory response to injury. Localized increases in the concentration of prostaglandins cause an increase in the inflammatory response, which speeds the healing process. The COX enzymes are classified as COX-1 and COX-2 specific for genes that express both enzymes. COX-1 is constitutively expressed, whereas COX-2 is an induced enzyme. In the case of microneedle-treated skin, COX-2 is expressed as a result of damage to skin.

Inhibition of the COX enzymes reduces the production of prostaglandins. Non-steroidal anti-inflammatory drugs (NSAIDS) have long been used to inhibit COX enzymes and reduce the inflammatory response to an injury. As described in detail below, NSAIDS have been classified based on whether they inhibit COX-1 or COX-2 (specific) or both (*i.e*., non-specific inhibitors). WO 03/002094 discloses the intradermal application of COX inhibitors with microneedle arrays.

It has now been found that the administration of COX inhibitors in conjunction with microneedles increases the duration in which the microneedle-created pore will remain open.

### SUMMARY

The embodiments described herein include the use of an agent (*e*.*g*., a COX inhibitor) to enhance the viability of microneedle-created pores when a microneedle array is used in conjunction with the transdermal administration of an active pharmaceutical agent, which may or may not be a COX inhibitor. The invention may be defined by the appendant claims.

### BRIEF DESCRIPTION OF THE DRAWINGS

Figure 1 shows the transepidermal water loss as a function of time for microneedle treated skin with a placebo and placebo alone in subject 1.

Figure 2 shows the transepidermal water loss as a function of time for microneedle-treated skin with a placebo and placebo alone in subject 2.

Figure 3 shows the transepidermal water loss as a function of time for microneedle-treated skin with a 3% celecoxib gel and celecoxib gel alone in subject 1.

Figure 4 shows the transepidermal water loss as a function of time for microneedle-treated skin with a 3% celecoxib gel and celecoxib gel alone in subject 2.

Figure 5 shows the transepidermal water loss as a function of time for microneedle-treated skin with a 3% diclofenac gel (Solaraze®), 3% diclofenac gel (Solaraze®) alone, microneedle with occlusion, and untreated skin.

Figure 6 shows the transepidermal water loss as a function of time for microneedle-treated skin with a 3% diclofenac gel (Solaraze®) and 3% diclofenac gel (Solaraze®) alone in subject 1.

Figure 7 shows the transepidermal water loss as a function of time for microneedle-treated skin with a 3% diclofenac gel (Solaraze®) and 3% diclofenac gel (Solaraze®) alone in subject 2.

Figure 8 shows the cascade of cellular and genetic events associated with the biosynthesis of prostaglandins from arachidonic acid. Figure 8 is a modified figure originally appearing in L.F. Fecker et al., The role of apoptosis in therapy and prophylaxis of epithelial tumours by nonsteroidal anti-inflammatory drugs (NSAIDs), 156 Suppl. 3 British J. Derm. 25-33 (2007).

### DESCRIPTION

The headings used throughout this disclosure are provided for convenience only and are not to be construed to limit the claims in any way. Embodiments illustrated under any heading may be combined with embodiments illustrated under any other heading.

As used herein, the term "pore viability" refers to pores, holes or channels created by the entry of one or more microneedles into the skin of a mammal in need of transdermal administration of an active pharmaceutical agent and the duration of lifetime that the resulting pores remain sufficiently open or "un-healed" thereby allowing the transdermal delivery of an active pharmaceutical agent to be systemically or locally delivered, whereby the dosing interval between microneedle treatments can be extended.

One embodiment described herein includes a drug delivery system, which includes a first COX inhibitor, to be used in conjunction with an array of microneedles which are arranged to penetrate the skin of a mammal in need of transdermal delivery of an active pharmaceutical agent for treating a medical condition. The drug delivery system described herein also includes an active pharmaceutical agent to be transdermally delivered to a mammal to treat a medical condition. The active pharmaceutical agent needed for treating a medical condition is different from the first COX inhibitor. In other embodiments, the COX inhibitor is a COX-1 inhibitor. In a further embodiment, the COX inhibitor is a COX-2 inhibitor. In another embodiment, the COX inhibitor is both a COX-1 and a COX-2 inhibitor (*i.e*., a non-specific COX inhibitor). In a further embodiment the COX inhibitor can be in any pharmaceutically acceptable form (*e.g*., salts, esters, prodrugs, etc.).

Also described herein are compositions comprising a first COX inhibitor which is useful in enhancing pore viability when used in conjunction with microneedle-treated skin. The compositions further comprise a first active pharmaceutical agent to be transdermally delivered to a mammal. The first COX inhibitor is different from the active pharmaceutical agent. In a further embodiment, the compositions further comprise a first pharmaceutical excipient. In another embodiment, the COX inhibitor is a COX-1 inhibitor. In a further embodiment, the COX inhibitor is a COX-2 inhibitor and in another embodiment, the COX inhibitor is both a COX-1 and a COX-2 inhibitor. In a further embodiment the COX inhibitor can be in any pharmaceutically acceptable form (e.g., salts, esters, prodrugs, etc.). In a further embodiment, the active pharmaceutical agent is a COX inhibitor. The active pharmaceutical agent is a COX inhibitor other than the COX inhibitor that is administered to enhance pore viability when used in conjunction with a microneedle-treated skin. In another embodiment, the active pharmaceutical agent is not a COX inhibitor.

Also described herein are methods of treating one or more medical conditions in a mammal by transdermally delivering an active pharmaceutical agent through pores created by microneedle treatment of the skin and which have been kept viable by the topical use of a COX inhibitor in conjunction with the microneedle treatment of the mammal's skin. In another embodiment, the COX inhibitor is a COX-1 inhibitor. In a further embodiment, the COX inhibitor is a COX-2 inhibitor and in another embodiment, the COX inhibitor is both a COX-1 and a COX-2 inhibitor. In a further embodiment, the COX inhibitor can be in any pharmaceutically acceptable form (*e.g*., salts, esters, prodrugs, etc.).

Further embodiments described herein include methods for enhancing or prolonging microneedle pore viability and comprise the use of a microneedle array to create pores in the skin of a mammal and topically applying a first COX inhibitor wherein the microneedle pores have an increased viability so that the rate and extent of transdermal delivery of an active pharmaceutical agent is increased. The first COX inhibitor is different from the active pharmaceutical agent. In another embodiment, the COX inhibitor is a COX-1 inhibitor. In a further embodiment, the COX inhibitor is a COX-2 inhibitor and in another embodiment, the COX inhibitor is both a COX-1 and a COX-2 inhibitor. In a further embodiment the COX inhibitor can be in any pharmaceutically acceptable form (*e.g*., salts, esters, prodrugs, etc.).

**Microneedle Array**

The compounds and pharmaceutical compositions described herein are suitable for use in conjunction with microneedles for transdermal drug delivery which create micrometer-scale transport pathways. Microneedles provide a minimally invasive means to transport molecules into and/or through the skin for local or systemic delivery of an active pharmaceutical agent. The channels or pores created by a microneedle array are extremely small on a clinical level. However, because the channels or pores are orders of magnitude larger than even macromolecules, such channels or pores have been shown to significantly increase skin permeability.

Microneedles can be can be solid or hollow and are made from many bio-compatible materials, including silicon, biodegradable polymers, and stainless steel. Solid microneedles can be used to create channels or pores in the skin, followed by application of a transdermal patch to the skin surface. Alternatively, solid microneedles can be first coated with an active pharmaceutical agent and then inserted into the skin. Hollow microneedles can also be used to facilitate active permeation through the bore in the microneedle and into the skin. *See*, *e.g*., Prausnitz, Microneedles for transdermal drug delivery, Adv. 56 Drug. Deliv. Rev. 581-587 (2004), for a review of some of the microneedle technology suitable for use with the various embodiments of the claimed invention described herein.

Numerous studies have demonstrated that solid microneedles can increase skin permeability by up to four orders of magnitude for compounds ranging in size from small molecules to proteins to nanoparticles. Henry et al., Microfabricated microneedles: a novel approach to transdermal drug delivery, 87 J. Pharm. Sci. 922-925 (1998); McAllister et al., Microfabricated needles for transdermal delivery of macromolecules and nanoparticles: fabrication methods and transport studies, 100 Proc. Nat'l Acad. Sci. 13755-13760 (2003); Lin et al., Transdermal delivery of antisense oligonucleotides with microprojection patch (Macroflux) technology, 18(12) Pharm. Res. 1787-1793 (2001); and Cormier et al., Transdermal delivery of desmopressin using a coated microneedle array patch system, 97 J. Control. Release. 503-511 (2004). Hollow microneedles have also been shown to deliver macromolecules such as insulin. *See* McAllister, Proc. Nat'l Acad. Sci. 13755-13760; Martanto et al., Transdermal delivery of insulin using microneedles in vivo, 21 Pharm. Res. 947-952 (2004). Microneedle insertion in human volunteers resulted in a sensation described as that similar to a smooth surface applied to the skin or the "sensation of a piece of tape" applied to the skin. Kaushik et al., Lack of pain associated with microfabricated microneedles, 92 Anesth. & Analg. 502-504 (2001).

Suitable microneedle arrangements for use with the compounds and compositions described herein can be found in the foregoing references as well as in United States Patent Application No. 11/812,249, published as US 2008-0008745 A1 on January 10, 2008.

Solid microneedle adhesive patches can be fabricated for insertion into the skin. Fixed microneedle geometries can be cut into 75 µm thick stainless steel sheets (Trinity Brand Industries, SS 304; McMaster-Carr, Atlanta, GA, USA) using an infrared laser (Resonetics Maestro, Nashua, NH, USA) and then can be manually bent perpendicular to the plane of their metal substrate. For better insertion and adhesion of patches to the skin, microneedle arrays can be assembled into adhesive patches. The adhesive would serve to hold the microneedles firmly against the skin by compensating for the mechanical mismatch between the flexible skin tissue and the rigid microneedle substrate. The microneedle patches can be assembled in a laminar flow hood for cleanliness and then sterilized using ethylene oxide (AN 74j, Andersen Sterilizers, Haw River, NC, USA) before use.

Microneedle arrays can be fabricated to produce patches containing 50 microneedles arranged in a 5 x 10 array of microneedles. Suitable individual microneedles can be about 620 µm in length, about 160 µm in width at the base, and less than about 1 µm in radius of curvature at the tip.

**COX Inhibitor**

The term COX inhibitor as used herein refers to compounds which prevent or reduce prostaglandin biosynthesis in mammals by inhibiting the production of prostaglandin G/H which is also know as cyclooxygenase or COX. There are two forms of cyclooxygenase, cyclooxygenase-1 (COX-1) and cyclooxygenase-2 (COX-2). As used herein, the term COX inhibitor includes those compounds which (i) primarily or exclusively inhibit the COX-1 enzyme; (ii) primarily or exclusively inhibit the COX-2 enzyme; and (iii) those compounds which inhibit both the COX-1 and COX-2 enzymes (*i.e*., non-specific inhibitors). The current understanding is that the COX inhibitor serves to enhance pore viability by slowing the healing process and also serves to reduce local inflammation caused by either (i) the administration of the microneedles, (ii) the formulation or (iii) the active pharmaceutical agent to be delivered to the patient.

Examples of COX inhibitors suitable for use in the various embodiments described herein include: aspirin, diflunisal, olsalazine, salsalate, sulfasalazine, acetaminophen, indomethacin, sulindac, etodolac, mefenamic acid, meclofenamate, flufenamic acid, tolmetin, ketorolac, diclofenac, ibuprofen, naproxen, fenoprofen, ketoprofen, flurbiprofen, oxaprozin, piroxicam, meloxicam, nabumetone, celecoxib, valdecoxib, rofecoxib, parecoxib, etoricoxib, lumiracoxib, valdecoxib, nimesulide, mofezolac, SC-560, FR122047, and DuP-697. Additional COX inhibitors can be found in Merck Index, Thirteenth Ed., The Physicians Desk Reference, 58^{th} Ed., and Goodman and Gilmans, "The Pharmacological Basis of Therapeutics, 11th Ed.

Pharmaceutically acceptable forms of a COX inhibitor include those which are suitable for transdermal administration to a mammal. The COX inhibitors described herein may be in any form suitable for administration to a mammal, such as in the form of a free base, free acid, salt, ester, hydrate, anhydrate, enantiomer, isomer, tautomer, polymorph, derivative, or the like.

Pharmaceutically acceptable salts of a COX inhibitor include salts suitable for administration to a mammal and includes those prepared from formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic, methanesulfonic, ethanesulfonic, benzenesulfonic, pantothenic, toluenesulfonic, 2-hydroxyethanesulfonic, sulfanilic, cyclohexylaminosulfonic, beta-hydroxybutyric, galactaric and galacturonic acids. The following list of pharmaceutically acceptable salts is not meant to be exhaustive but merely illustrative as person of ordinary skill in the art would appreciate that other pharmaceutically acceptable salts of a COX inhibitor may be prepared.

Acid addition salts can be prepared from the free base forms through a reaction of the free base with a suitable acid. Suitable acids for preparing acid addition salts include both organic acids, *e.g*., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like, as well as inorganic acids, *e.g*., hydrochloric acid, hydrobromic acid, sulfuric acid, nitric acid, phosphoric acid, and the like. The following list of organic and inorganic acids is not meant to be exhaustive but merely illustrative as person of ordinary skill in the art would appreciate that other acids may be used to create pharmaceutically acceptable salts of a COX inhibitor. In other embodiments, an acid addition salt is reconverted to the free base by treatment with a suitable base. In still other embodiments, the basic salts are alkali metal salts, *e.g*., sodium salt.

**Active Pharmaceutical Agent**

As used herein, the term "active pharmaceutical agent" is an agent, including any chemical, protein, peptide, or nucleotide, that is administered to a mammal in order to achieve a desired therapeutic result. Examples of active pharmaceutical agents can be found in The Merck Index: An Encyclopedia of Chemicals, Drugs, and Biologicals, 14th Edition; Goodman & Gilman's The Pharmacological Basis of Therapeutics, 11th Edition; Physicians Desk Reference 2008, 62 Edition; Remington's Pharmaceutical Sciences, 16th Edition, In one embodiment, the active pharmaceutical agent is a COX inhibitor. The COX inhibitor administered to maintain the viability of the microneedle-created pore opening is different than the COX inhibitor administered as the active pharmaceutical agent. In a further embodiment, the active pharmaceutical agent is an agent other than a COX inhibitor.

In an embodiment, the active pharmaceutical agent is administered to achieve a systemic therapeutic result. In one embodiment, the compositions and drug delivery systems described herein are suitable for the transdermal administration of an active pharmaceutical agent. In a further embodiment, the compositions and drug delivery systems described herein are suitable for transdermal administration of an active pharmaceutical agent in order to achieve a systemic therapeutic benefit. In an additional embodiment, the compositions and drug delivery systems described herein are suitable for topical administration of an active pharmaceutical agent. By way of example, and without limitation to the invention described herein, topical administration, can include, but is not limited to, administration to the epidermal and dermal regions of the skin. In a further embodiment, the compositions and drug delivery systems described herein are suitable for administration of an active pharmaceutical agent in order to achieve a localized therapeutic benefit. By way of example, and without limitation to the invention described herein, topical therapeutic effects, can include, but are not limited to, therapeutic effects in the subcutaneous, muscular and joint regions near the area of administration. In another embodiment, the compositions and systems described herein are adapted for use in or on the abdomen, back, chest, legs, arms, scalp or other suitable skin surface and may be formulated as microneedle-containing patches to be used in conjunction with a microneedle array or other forms suitable for use in conjunction with a microneedle array such as ointments, creams, suspensions, lotions, pastes, gels, sprays, foams or oils which can be optionally occluded.

In another embodiment, a single dosage unit comprises a first COX inhibitor and a therapeutically effective amount or a therapeutically and/or prophylactically effective amount of an active pharmaceutical agent. The term "therapeutically effective amount" or "therapeutically and/or prophylactically effective amount" as used herein refers to an amount of compound or agent that is sufficient to elicit the required or desired therapeutic and/or prophylactic response, as the particular treatment context may require. Single dosage unit as used herein includes individual patches which incorporate at least a first COX inhibitor which can be applied after the skin has been treated with a microneedle array which forms pores through which an active pharmaceutical agent can be delivered.

It will be understood that a therapeutically and/or prophylactically effective amount of a drug for a subject is dependent *inter alia* on the body weight of the subject as well as other factors known to a person of ordinary skill in the art. A "subject" herein to which a therapeutic agent or composition thereof can be administered includes mammals such as a human subject of either sex and of any age, and also includes any nonhuman animal, particularly a domestic, farm or companion animal, illustratively a cat, cow, pig, dog or a horse as well as laboratory animals such as guinea pigs and primates.

In another embodiment, compositions disclosed herein comprise a first active pharmaceutical agent in a total amount between about 0.1% and about 95% by weight of the composition which is transdermally administrable, for example about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 4%, about 5%, about 6%, about 7%, about 8%, about 9%, about 10%, about 11%, about 12%, about 13%, about 14%, about 15%, about 20%, about 25%, about 30%, about 35%, about 40%, about 45%, about 50%, about 55%, about 60%, about 65%, about 70%, about 75%, about 80%, about 85%, about 90% or about 95%.

The active pharmaceutical agents that can be transdermally administered using the methods described herein are not limited. Indeed, traditional limitations or constraints placed upon the transdermal administration of an active pharmaceutical agent such as the molecular weight or size, molecular charge or water/octanol partition coefficients are not limiting factors when the active pharmaceutical agent is administered with microneedles and a COX inhibitor as described herein. Indeed, very large molecules such as proteins, antibodies, peptides, DNA, vaccines and nanoparticles, which would otherwise typically be considered unsuitable for other transdermal passive diffusion systems, can be delivered through the skin for local and/or systemic action. Additionally, active pharmaceutical agents which are water soluble have traditionally been thought to be unsuitable for transdermal delivery due to the lipid nature of the stratum corneum. However, with the use of the microneedle and COX inhibitor as described herein, even highly water soluble compounds can be transdermally delivered via the pores created by microneedle treatment of the skin. Thus, nearly any type or class of active pharmaceutical agent can be delivered in conjunction with a microneedle treatment of the skin and use of a COX inhibitor as described herein.

**Methods of Treating a Medical Condition**

Disclosed are methods of treating one or more medical conditions in a a mammal by transdermally delivering an active pharmaceutical agent through the pores created by a microneedle array which has penetrated the skin of a mammal and the resulting pores have been kept suitably viable for the delivery of an active pharmaceutical agent by the topical use of a COX inhibitor in conjunction with the use of a microneedle array. The COX inhibitor can be a COX-1 inhibitor, a COX-2 inhibitor or an inhibitor of both COX-1 and COX-2. The COX inhibitor can be in any pharmaceutically acceptable form. The medical condition to be treated is not limited and can be any condition for which the active pharmaceutical agent required for treatment can be delivered transdermally by use of a microneedle array and a first COX inhibitor as described herein. By way of example, and without limitation to the invention described herein, the medical condition to be treated can include: pain disorders, cardiovascular disorders, respiratory disorders, gastrointestinal disorders, renal disorders, psychiatric disorders, endocrinological, gynecological and obstetric disorders, immunological disorders, bone and joint disorders, hematological disorders, oncologic disorders, nutritional disorders, and infectious diseases. The use of specific active pharmaceutical agents to treat the exemplified disorders is known in the skill in the art, as exemplified in The Physicians Desk Reference, 58th Ed., and Goodman and Gilmans, "The Pharmacological Basis of Therapeutics, 11th Ed.

The terms "treat", "treated", "treating" and "treatment" are to be broadly understood as referring to any response to, or anticipation of, a medical condition in a mammal, particularly a human, and includes but is not limited to:
(i) preventing the medical condition from occurring in a subject, which may or may not be predisposed to the condition, but has not yet been diagnosed with the condition and, accordingly, the treatment constitutes prophylactic treatment for the medical condition;
(ii) inhibiting the medical condition, i.e., arresting, slowing or delaying the onset, development or progression of the medical condition; or
(iii) relieving the medical condition, i.e., causing regression of the medical condition.

**Pharmaceutical Excipients**

The pharmaceutical compositions and drug delivery systems described herein can, if desired, include one or more pharmaceutically acceptable excipients. The term "excipient" herein means any substance, not itself an active pharmaceutical agent, used in conjunction with the active pharmaceutical agent delivered to a subject or added to a pharmaceutical composition or drug delivery system to improve one of more characteristics, such as its handling or storage properties or to permit or facilitate formation of a dose unit of the composition. Excipients include, by way of illustration and not limitation, solvents, thickening agents, penetration enhancers, wetting agents, lubricants, emollients, substances added to mask or counteract a disagreeable odor or flavor, fragrances, and substances added to improve appearance or texture of the composition or drug delivery system. Any such excipients can be used in any dosage forms of the present disclosure. The foregoing list of excipients is not meant to be exhaustive but merely illustrative as a person of ordinary skill in the art would recognize that additional excipients could be utilized.

The compositions and drug delivery systems described herein containing excipients can be prepared by any technique known to a person of ordinary skill in the art of pharmacy, pharmaceutics, drug delivery, pharmacokinetics, medicine or other related discipline that comprises admixing one or more excipients with a therapeutic agent to form a composition, drug delivery system or component thereof.

Non-limiting examples of penetration enhancing agents include C8-C22 fatty acids such as isostearic acid, octanoic acid, and oleic acid; C8-C22 fatty alcohols such as oleyl alcohol and lauryl alcohol; lower alkyl esters of C8-C22 fatty acids such as ethyl oleate, isopropyl myristate, butyl stearate, and methyl laurate; di(lower)alkyl esters of C6-C22 diacids such as diisopropyl adipate; monoglycerides of C8-C22 fatty acids such as glyceryl monolaurate; tetrahydrofurfuryl alcohol polyethylene glycol ether; polyethylene glycol, propylene glycol; 2-(2-ethoxyethoxy)ethanol; diethylene glycol monomethyl ether; alkylaryl ethers of polyethylene oxide; polyethylene oxide monomethyl ethers; polyethylene oxide dimethyl ethers; dimethyl sulfoxide; glycerol; ethyl acetate; acetoacetic ester; N-alkylpyrrolidone; and terpenes. Additional penetration enhancers suitable for use can also be found in United States Pat. App. No. 10/032,163, published as US 2002-0111377 A1 on August 15, 2002.

The thickening agents (aka gelling agents) used herein may include anionic polymers such as polyacrylic acid (CARBOPOL® by Noveon, Inc., Cleveland, Ohio), carboxypolymethylene, carboxymethylcellulose and the like, including derivatives of Carbopol® polymers, such as Carbopol® Ultrez 10, Carbopol® 940, Carbopol® 941, Carbopol® 954, Carbopol® 980, Carbopol® 981, Carbopol® ETD 2001, Carbopol® EZ-2 and Carbopol® EZ-3, and other polymers such as Pemulen® polymeric emulsifiers, and Noveon® polycarbophils. Additional thickening agents, enhancers and adjuvants may generally be found in Remington's The Science and Practice of Pharmacy as well as the Handbook of Pharmaceutical Excipients, Arthur H. Kibbe ed. 2000. Thickening agents or gelling agents are present in an amount sufficient to provide the desired rheological properties of the composition. Illustratively, one or more pharmaceutically acceptable thickening agent or gelling agent are present in a total amount by weight of about 0.1%, about 0.25%, about 0.5%, about 0.75%, about 1%, about 1.25%, about 1.5%, about 1.75%, about 2.0%, about 2.25%, about 2.5%, about 2.75%, about 3.0%, about 3.25%, about 3.5%, about 3.75%, about 4.0%, about 4.25%, about 4.5%, about 4.75%, about 5.0%, about 5.25%, about 5.5%, about 5.75%, about 6.0%, about 6.25%, about 6.5%, about 6.75%, about 7.0%, about 7.25%, about 7.5%, about 7.75%, about 8.0%, about 8.25%, about 8.5%, about 8.75%, about 9.0%, about 9.25%, about 9.5%, about 9.75%, about 10%, about 11%, about 11.5%, about 12%, about 12.5%, about 13%, about 13.5%, about 14%, about 14.5% or about 15%.

In one embodiment a neutralizing agent is optionally present to assist in forming a gel. Suitable neutralizing agents include sodium hydroxide (e.g., as an aqueous mixture), potassium hydroxide (e.g., as an aqueous mixture), ammonium hydroxide (e.g., as an aqueous mixture), triethanolamine, tromethamine (2-amino 2-hydroxymethyl-1, 3 propanediol), aminomethyl propanol (AMP), tetrahydroxypropyl ethylene diamine, diisopropanolamine, Ethomeen C-25 (Armac Industrial Division), Di-2 (ethylhexyl) amine (BASF-Wyandotte Corp., Intermediate Chemicals Division), triamylamine, Jeffamine D-1000 (Jefferson Chemical Co.), b-Dimethylaminopropionitrite (American Cyanamid Co.), Armeen CD (Armac Industrial Division), Alamine 7D (Henkel Corporation), dodecylamine and morpholine. The neutralizing agent is present in an amount sufficient to form a gel which is suitable for contact with the skin of a mammal.

In a further embodiment, the formulation is a gel, an ointment, a cream or a patch and comprises a pharmaceutically active agent, optionally one or more penetration enhancing agent, thickening agent, lower alcohol, such as ethanol or isopropanol, or water. In another embodiment, the formulation is a gel, an ointment, a cream or a patch, further comprised of sodium hydroxide or triethanolamine or potassium hydroxide, or a combination thereof, in an amount sufficient, as is known in the art, to assist the gelling agent in forming a gel suitable for contact with the skin of a mammal.

Compositions described herein optionally comprise one or more pharmaceutically acceptable wetting agents as excipients. Non-limiting examples of surfactants that can be used as wetting agents in compositions of the disclosure include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (*e.g*., Labrasol™ of Gattefossé), polyoxyethylene (35) castor oil and polyoxyethylene (40) hydrogenated castor oil, polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e.g.*, Tween™ 80 of ICI), propylene glycol fatty acid esters, for example propylene glycol laurate (*e.g*., Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, about 0.4% to about 10%, or about 0.5% to about 5%, of the total weight of the composition. Illustratively, one or more pharmaceutically acceptable wetting agents are present in a total amount by weight of about 0.25%, about 0.5%, about 0.75%, about 1%, about 1.25%, about 1.5%, about 1.75%, about 2.0%, about 2.25%, about 2.5%, about 2.75%, about 3.0%, about 3.25%, about 3.5%, about 3.75%, about 4.0%, about 4.25%, about 4.5%, about 4.75%, about 5.0%, about 5.25%, about 5.5%, about 5.75%, about 6.0%, about 6.25%, about 6.5%, about 6.75%, about 7.0%, about 7.25%, about 7.5%, about 7.75%, about 8.0%, about 8.25%, about 8.5%, about 8.75%, about 9.0%, about 9.25%, about 9.5%, about 9.75% or about 10%.

Compositions described herein optionally comprise one or more pharmaceutically acceptable lubricants (including anti-adherents and/or glidants) as excipients. Suitable lubricants include, either individually or in combination, glyceryl behapate (*e.g*., Compritol™ 888); stearic acid and salts thereof, including magnesium (magnesium stearate), calcium and sodium stearates; hydrogenated vegetable oils (*e.g*., Sterotex™); colloidal silica; talc; waxes; boric acid; sodium benzoate; sodium acetate; sodium fumarate; sodium chloride; DL-leucine; PEG (*e.g*., Carbowax™ 4000 and Carbowax™ 6000); sodium oleate; sodium lauryl sulfate; and magnesium lauryl sulfate. Such lubricants, if present, constitute in total about 0.1% to about 10%, about 0.2% to about 8%, or about 0.25% to about 5%, of the total weight of the composition. Illustratively, one or more pharmaceutically acceptable lubricants are present in a total amount by weight of about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1.5%, about 1.6%, about 1.7%, about 1.8%, about 1.9%, about 2.0%, about 2.1%, about 2.2%, about 2.3%, about 2.4%, about 2.5%, about 2.6%, about 2.7%, about 2.8%, about 2.9%, about 3.0%, about 3.1 %, about 3.2%, about 3.3%, about 3.4%, about 3.5%, about 3.6%, about 3.7%, about 3.8%, about 3.9%, about 4.0%, about 4.1%, about 4.2%, about 4.3%, about 4.4%, about 4.5%, about 4.6%, about 4.7%, about 4.8%, about 4.9%, about 5.0%, about 5.1%, about 5.2%, about 5.3%, about 5.4%, about 5.5%, about 5.6%, about 5.7%, about 5.8%, about 5.9%, about 6.0%, about 6.1%, about 6.2%, about 6.3%, about 6.4%, about 6.5%, about 6.6%, about 6.7%, about 6.8%, about 6.9%, about 7.0%, about 7.1%, about 7.2%, about 7.3%, about 7.4%, about 7.5%, about 7.6%, about 7.7%, about 7.8%, about 7.9%, about 8.0%, about 8.1%, about 8.2%, about 8.3%, about 8.4%, about 8.5%, about 8.6%, about 8.7%, about 8.8%, about 8.9%, about 9.0%, about 9.1%, about 9.2%, about 9.3%, about 9.4%, about 9.5%, about 9.6%, about 9.7%, about 9.8%, about 9.9% or about 10.0%.

In another embodiment, the compositions described herein optionally comprise an emollient. Illustrative emollients include mineral oil, mixtures of mineral oil and lanolin alcohols, cetyl alcohol, cetostearyl alcohol, petrolatum, petrolatum and lanolin alcohols, cetyl esters wax, cholesterol, glycerin, glyceryl monostearate, isopropyl myristate, isopropyl palmitate, lecithin, allyl caproate, althea officinalis extract, arachidyl alcohol, argobase EUC, Butylene glycol dicaprylate/dicaprate, acacia, allantoin, carrageenan, cetyl dimethicone, cyclomethicone, diethyl succinate, dihydroabietyl behenate, dioctyl adipate, ethyl laurate, ethyl palmitate, ethyl stearate, isoamyl laurate, octanoate, PEG-75 lanolin, sorbitan laurate, walnut oil, wheat germ oil super refined almond, super refined sesame, super refined soybean, octyl palmitate, caprylic/capric triglyceride and glyceryl cocoate.

An emollient, if present, is present in the compositions described herein in an amount of about 1% to about 30%, about 3% to about 25%, or about 5% to about 15%, by weight. Illustratively, one or more emollients are present in a total amount by weight of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, or about 30%.

In one embodiment, a composition comprises an antimicrobial preservative. Illustrative anti-microbial preservatives include acids, including but not limited to benzoic acid, phenolic acid, sorbic acids, alcohols, benzethonium chloride, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, propylparaben, sodium propionate, or thimerosal. The anti-microbial preservative, if present, is present in an amount of about 0.1% to about 5%, about 0.2% to about 3%, or about 0.3% to about 2%, by weight, for example about 0.2%, 0.4%, 0.6%, 0.8%, 1%, 1.2%, 1.4%, 1.6%, 1.8%, 2%, 2.4%, 2.6%. 2.8%, 3.0%, 3.2%, 3.4%, 3.6%, 3.8%, 4%, 4.2%, 4.4%, 4.6%, 4.8%, or 5%.

Compositions described herein optionally compromise one or more emulsifying agents. The term "emulsifying agent" refers to an agent capable of lowering surface tension between a non-polar and polar phase and includes compounds defined as "self-emulsifying" agents. Suitable emulsifying agents can come from any class of pharmaceutically acceptable emulsifying agents including carbohydrates, proteins, high molecular weight alcohols, wetting agents, waxes and finely divided solids. The optional emulsifying agent, if present, is present in a composition in a total amount of about 1% to about 15%, about 1% to about 12%, about 1% to about 10%, or about 1% to about 5% by weight of the composition. Illustratively, one or more emulsifying agents are present in a total amount by weight of about 1%, about 2%, about 3%, about 4%, about 5%, about 6%, about 7%, about 8%, about 9%, about 10%, about 11%, about 12%, about 13%, about 14%, or about 15%.

In another embodiment, the water immiscible solvent comprises propylene glycol, and is present in a composition in an amount of about 1% to about 99%, by weight of the composition, for example about 1%, about 2%, about 3%, about 4%, about 5%, about 6%, about 6%, about 7%, about 8%, about 9%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95% or about 99%.

**Pharmaceutical Dosage Forms**

As used herein, a "transdermal drug delivery system" comprises a first array of microneedles used in conjunction with a first COX inhibitor and an active pharmaceutical agent. Various alternative embodiments of the invention described and claimed herein include (i) the use of a COX inhibitor-containing gel that could be applied to the skin surface either before, during, or after the skin had been treated with a microneedle array such as a microneedle array device; (ii) a patch comprising a COX inhibitor and an active pharmaceutical agent could be applied to the skin surface after the skin had been treated with a microneedle array such as a microneedle array device (iii) an patch application could be incorporated with a microneedle array application device; (iv) the COX inhibitor could be part of a microneedle coating; and (v) the COX inhibitor could be part of a formulation which is delivered through hollow microneedles and into the skin. Examples of microneedle array devices include http://www.macroflux.com/b1.htm and http://www.bd.com/technologies/add/#PlatformTechnology.

In one embodiment, the compounds and pharmaceutical compositions described herein are suitable for use in transdermal delivery systems such as a patch to be used in conjunction with a microneedle array. For example, the compounds and compositions described herein are suitable for use in a membrane-modulated transdermal delivery system. In one embodiment of this system, the reservoir containing the active pharmaceutical agent to be transdermally administered to the patient can be encapsulated in a shallow compartment molded from a drug impermeable backing and a rate controlling polymeric membrane through which the compound to be delivered passes in a controlled manner. In another embodiment, the external surface of the membrane has a thin layer of a drug-compatible, hypoallergenic adhesive polymer (e.g., silicone or polyacrylate adhesive) which is applied to achieve intimate contact of the transdermal system with the skin.

The compounds and pharmaceutical compositions described herein are also suitable for use in adhesive-diffusion controlled transdermal systems in conjunction with a microneedle array. In these embodiments, the drug reservoir can be formulated by directly dispersing the active pharmaceutical agent (or agents) to be delivered in an adhesive polymer and then spreading the medicated adhesive onto a flat sheet of drug-impermeable backing membrane to form a thin active pharmaceutical agent reservoir layer. Optionally, on top of the drug reservoir layer, additional layers of non-medicated rate controlling adhesive polymer of constant thickness are placed to produce an adhesive diffusion-controlled drug-delivery system. The resulting adhesive-diffusion controlled transdermal system is then applied to the area of the skin which has previously undergone microneedle treatment.

The compounds and pharmaceutical compositions described herein are also suitable for use in matrix dispersion-type systems in conjunction with microneedle arrays. In these systems, the reservoir containing the active pharmaceutical agent (or agents) can be formed by homogeneously dispersing the active pharmaceutical agent (or agents) in a hydrophilic or lipophilic polymer matrix, and the medicated polymer then is molded into a medicated disc with a defined surface area and controlled thickness. The disc then is glued onto an occlusive baseplate in a compartment fabricated from a drug-impermeable backing. The adhesive polymer is spread along the circumference to form a strip of adhesive rim around the medicated disc. The resulting dispersion-type transdermal system is then applied to the area of the skin which has previously undergone microneedle treatment.

The compounds and pharmaceutical compositions described herein are also suitable for use in microreservoir systems in conjunction with microneedle arrays. In these systems, the drug reservoir is formed by first suspending the drug particles in an aqueous solution of watersoluble polymer and then dispersing it homogeneously in a lipophilic polymer by high-shear mechanical force to form a large number of unleachable, microscopic spheres reservoirs of active pharmaceutical agent (or agents). This unstable dispersion is quickly stabilized by immediately cross-linking the polymer which produces a medicated polymer disc with a constant surface area and fixed thickness. A transdermal therapeutic system is produced in which the medicated disc is positioned at the center and surrounded by an adhesive rim. The resulting microreservoir transdermal system is then applied to the area of the skin which has previously undergone microneedle treatment.

In one embodiment, the drug and adhesive are formulated into one monolithic layer. The drug can be mixed with an adhesive (e.g. silicone type, available from Dow Coming and other manufacturers) in a solvent (e.g. methylene chloride or ethyl acetate). This drug mixture would then be extruded onto a polyester backing film to a uniform thickness of about 100 microns or greater with a precision wet-film applicator. The solvent is allowed to evaporate in a drying oven and the resulting "patch" is trimmed to the appropriate size. Various patch formulations will be made until the desired steady-state flux rate and adhesive properties are obtained. Different adhesives can be tried, as well as varying the amount of adhesive in the formulation (Nalluri, Milligan et al. 2005). Suitable results have been obtained by making monolithic patches with DURO-TAK 387-2051, which is an acrylate-vinyl acetate non-curing pressure sensitive adhesive from the National Starch Chemical Company. Different solvents (e.g. isopropyl myristate, propylene glycol) can optionally be incorporated into the formulation in an attempt to optimize the delivery rate of the active pharmaceutical agent. In a further embodiment, reservoir patches can be made if it appears, for example, that the drugs are not compatible with a monolithic matrix patch formulation. In the reservoir system, the active ingredient(s) and any excipient(s) could be formulated into a gel and sealed between a release layer and an impermeable backing material such as polyester or other suitable material known to a person of skill in the art. Ethyl vinyl acetate membranes with acrylic adhesives have been found to be suitable. In each of the foregoing embodiments, the patch would then be applied to the area of the skin which has previously undergone microneedle treatment.

Adhesive patch formulations can be prepared containing different loadings of the active pharmaceutical agent (or agents) to be delivered transdermally by using DURO-TAK adhesives (National Starch and Chemical Company, USA). Appropriate amounts of adhesive and drug can be sonicated for ten minutes, cast onto the release liner (9742 Scotchpak, 3M, St. Paul, MN) with a wet film applicator (Paul N. Gardner Company, Inc., Pompano Beach, FL) set at a 40 mil thickness, and kept at room temperature for one hour and then at 70°C in an oven for ten minutes (to remove any residual solvent). The patches would then be covered with backing membrane (CoTran 9722, 3M, St. Paul, MN), will be cut into appropriate sizes, and then can be stored in a desiccator for further study. The resulting patches would then be applied to the area of the skin which has previously undergone microneedle treatment.

In further embodiments, additional adhesives which are suitable for preparing patch formulations and transdermal delivery systems such as patches include polyisobutylenes, acrylates, silicone and combinations of the foregoing. Additional adhesives can be found in United States Patent Application No.11/860,432, published as US 2008/0076789 on March 27, 2008.

The compounds and pharmaceutical compositions described herein are also suitable for use the use of a COX inhibitor-containing gel that could be applied to the skin surface either before, during or after the skin had been treated with a microneedle array. In a further embodiment, in addition to the COX inhibitor, the gel would also contain an active pharmaceutical agent.

In another illustrative embodiment, the transdermal patch incorporating a first COX inhibitor and an active pharmaceutical agent is applied to the area of the skin which has undergone microneedle treatment and is capable of controlling the release of the active pharmaceutical agent such that transdermal delivery of the active pharmaceutical agent to the subject is substantially uniform and sustained over a period of about 1 hour, about 2 hours, about 3 hours, about 4 hours, about 6 hours, about 12 hours, about 24 hours, about 48 hours, about 72 hours, about 96 hours, about 5 days, about 6 days or about 7 days. Such transdermal patch which can be used in the practice of the methods described herein can take the form of an occlusive body. In practice, the occlusive body which includes the first COX inhibitor and a first active pharmaceutical agent is applied to the area of the skin which has undergone microneedle treatment to transdermally deliver the active pharmaceutical agent.

**Methods of Enhancing Microneedle Pore Viability**

Methods for enhancing microneedle pore viability are described herein and comprise the use of a microneedle array to create pores in the skin of a mammal and topically applying a first COX inhibitor in conjunction with the use of the microneedle array wherein the resulting pores have enhanced viability so that the rate and extent of transdermal delivery of an active pharmaceutical agent is increased relative to the non-use of a COX inhibitor. In other embodiments the COX inhibitor can be a COX-1 inhibitor, a COX-2 inhibitor or an inhibitor of both COX-1 and COX-2. In a further embodiment the COX inhibitor can be in any pharmaceutically acceptable form. The COX inhibitor is different from the active pharmaceutical agent.

### EXAMPLES

**SECTION I. SUMMARY**

Examples that do not pertain to the scope of the claims serve for illustrative purposes only. The goal of the current study described herein was to evaluate pore viability enhancement of microneedle-created pores. COX specific and non-specific inhibitors were studied in order to determine if a decrease in prostaglandins would result in extended dosing interval after treatment with microneedles. COX inhibitors such as diclofenac and celecoxib showed enhanced viability of microneedle pores as compared to previous results which indicated a two-day delivery window. A placebo effect was not observed, therefore, the COX inhibitors may have contributed to inhibiting normal healing pathways by preventing synthesis of prostaglandins which are important for natural wound healing.

COX inhibitor from classes II, III, and VII shown in Table 1, Solaraze^{®} Gel (diclofenac sodium 3%), ketoprofen and ibuprofen 10% gel and 3% celecoxib gel, were tested alongside a placebo formulation with and without the exposure to microneedles. Diclofenac gel is commonly prescribed for treatment of actinic keratoses, as it has been shown to produce strong anti-neoplastic effects. Ibuprofen and ketoprofen are commonly used for pain and inflammation ranging from headaches, muscle aches and sprains. Celecoxib is prescribed for the treatment of inflammation associated with arthritis.

**SECTION II. METHODOLOGY**

**1.0 Purpose**

The purpose of this study is to evaluate any enhancement to the viability of microneedle-created pores with COX specific and non-specific inhibitors and determine if a decrease in prostaglandins would result in extended dosing interval after treatment with microneedles.

**2.0 COX inhibitors**

**Table 1**

| Chemical groups | COX inhibitors |
|---|---|
| I | Salicylates |
| II | Arylalkanoic acids |
| III | 2-arylpropionic acids (profens) |
| IV | N-arylanthranilic acids (fenamic) acids |
| V | Pyrazolidine derivatives |
| VI | Oxicams |
| VII | Coxibs |
| VIII | Sulphonanilides |

**Table 2**

| Chemical groups | COX-1 and COX-2 inhibitors² | Chemical groups | Selective COX-2 inhibitors² |
|---|---|---|---|
| I | Aspirin | II | Etodolac |
| I | Diflunisal | VI | Meloxicam |
| I | Olsalazine | VII | Celecoxib |
| I | Salsalate | VII | DuP-697 |
| I | Sulfasalazine | VII | Rofecoxib |
| II | Diclofenac | VIII | Nimesulide |
| II | Indomethacin | IX | Lumiracoxib |
| II | Ketorolac | | |
| II | Sulindac | | |
| III | Ibuprofen | | |
| III | Ketoprofen | | |
| III | Naproxen | | |
| VI | Piroxicam | | |

| | | | |
|---|---|---|---|
| Several important inhibitors (Modified from L.F. Fecker et al., The role of apoptosis in therapy and prophylaxis of epithelial tumours by non-steroidal anti-inflammatory drugs (NSAIDS), 156 Suppl. 3 British J Dermatology. 25-33 (2007).) | | | |

**3.0 Formulations**

Diclofenac gel (Solaraze^{®} Gel, Doak Dermatologics, Fairfield, NJ) was purchased and used in the commercial form along with a 3% gel prepared from bulk diclofenac. Ibuprofen, ketoprofen and celecoxib were all formulated as simple gels containing a 2% non-ionic polymer (Klucel^{®}, Hercules Inc, Wilmington, DE).

All NSAIDS could be prepared from 0.5% to 20% in gel formulations as an adjuvant to enhance microneedle permeation of the active pharmaceutical ingredient by enhancing the viability of microneedle created pores.

All gels were prepared by mixing thoroughly with vortexing and sonication for 30 min. No base addition was required to induce polymer relaxation as Klucel^{®} is a non-ionic polymer that swells naturally when wetted.

3.1 Solaraze^{®} Gel (Table 3)

| Ingredients |
|---|
| diclofenac sodium (30 mg/g) |
| hyaluronate sodium |
| benzyl alcohol |
| polyethylene glycol monomethyl ether |
| purified water |

3.2 10% Ketoprofen (Table 4)

| %w/w | Weight (mg) |
|---|---|
| 10% ketoprofen, USP | 300 |
| 2% Klucel^{®} | 60 |
| 60% polyethylene glycol 400 | 1800 |
| 28% ethanol, 200 proof, USP/NF | 840 |

3.3 10% Ibuprofen (Table 5)

| %w/w | Weight (mg) |
|---|---|
| 10% ibuprofen, USP | 300 |
| 2% Klucel^{®} | 60 |
| 60% polyethylene glycol 400 | 1800 |
| 28% ethanol, 200 proof, USP/NF | 840 |

3.4 3% Celecoxib (Table 6)

| %w/w | Weight (mg) |
|---|---|
| 3% celecoxib, USP | 90 |
| 2% Klucel^{®} | 60 |
| 60% polyethylene glycol 400 | 1800 |
| 35% ethanol, 200 proof, USP/NF | 1050 |

3.5 3% Diclofenac (Table 7)

| %w/w | Weight (mg) |
|---|---|
| 3% diclofenac, USP | 90 |
| 2% Klucel^{®} | 60 |
| 60% polyethylene glycol 400 | 1800 |
| 35% ethanol, 200 proof, USP/NF | 1050 |

3.6 Placebo formulation (Table 8)

| %w/w | Weight (mg) |
|---|---|
| 0% API | 0 |
| 2% Klucel^{®} | 60 |
| 60% polyethylene glycol 400 | 1800 |
| 38% ethanol, 200 proof, USP/NF | 1140 |

**4.0 Microneedle Studies**

Two trials were completed with subject 1 and one trial was completed with subject 2. During the initial trial, subject one was tested for 7 days with only Solaraze^{®} Gel. 100 microneedle arrays were placed on each test site and covered with gel. The gel was then protected with an occlusive backing membrane (3M™ Scotchpak™ 9733 2.05 mil polyester film, St. Paul, MN) and secured to the test site with Bioclusive* tape (Johnson and Johnson Medical Ltd, Gargrave, Skipton, UK). Along with gel test sites, control sites with and without microneedle insertions were tested minus gel. The control sites were treated in the same manner with gel minus the 100 microneedle insertions. The initial trial lasted for 7 days followed by a 5 day study.

In trial 2, the same procedure was performed on a different test and control sites using each of the following gel formulations: placebo, Solaraze^{®} Gel, 3% diclofenac, 3% celecoxib, 10% ibuprofen, 10% ketoprofen, and controls with and without microneedle treatment. Each day, the protective covering was removed, areas were blotted 3 times with a Kimwipe^{®} (Kimberly-Clark, Roswell, GA) to remove gel and trapped moisture and TEWL readings were made with an RG1 Evaporimeter (cyberDERM, Broomall, PA). The treated areas had the formula re-applied and immediately covered to prevent closure of the microneedle created pores.

**SECTION III. RESULTS**

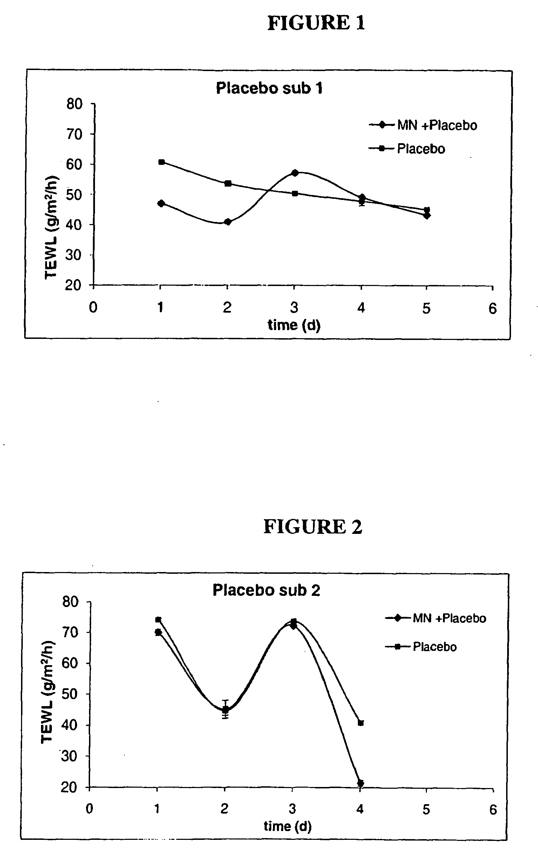

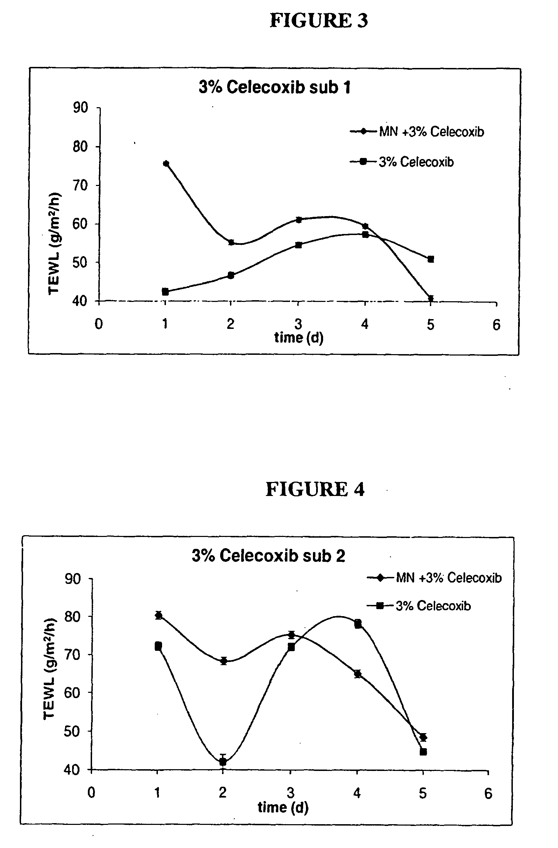

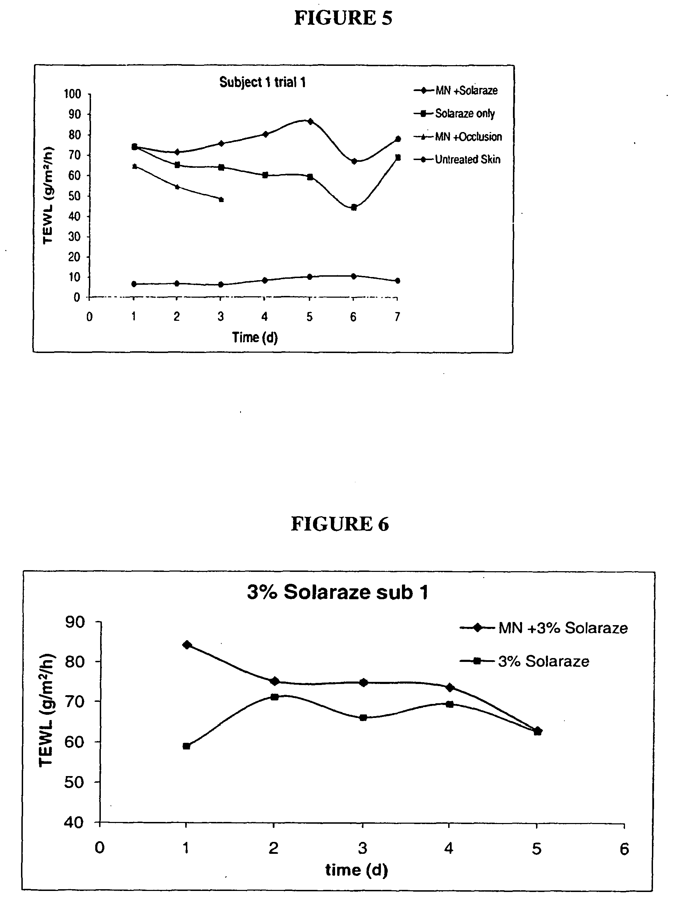

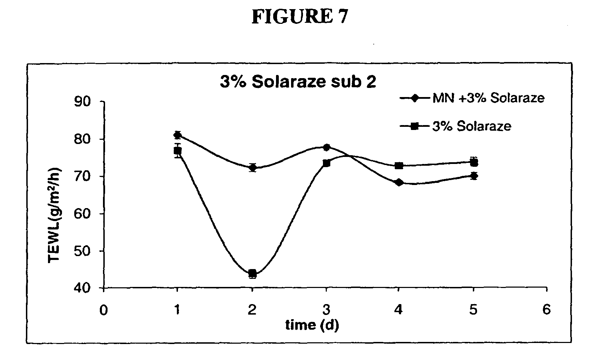

No enhancement in pore viability was observed between control and microneedle-treated areas of ketoprofen and ibuprofen. Similarly, the prepared formulation of 3% diclofenac was ineffective in increasing pore viability throughout the study. These ineffective examples may be the result of formulation design as permeability from the gel might not have been optimized. No effect was seen from the placebo formulation; therefore, any enhancement in pore lifetime was likely a result from the COX inhibitors. There was overlap in TEWL readings throughout the course of the 5 day study in both subjects as shown in Figures 1 and 2. However, significant enhancement (p<0.05) in TEWL readings were observed in subject 1 for 4 days and in subject 2 for 3 days in microneedle-treated skin with 3% celecoxib, depicted in Figures 3 and 4, respectively. The most significant pore viability enhancement came with Solaraze^{®} Gel as trial 1 showed over 7 days enhanced microneedle-treated skin with Solaraze^{®} Gel, as compared to Solaraze^{®} Gel alone, as shown in Figure 5. Also shown is a decreasing trend of microneedle occluded skin control over the first 3 days of the trial which is consistent with the closing of microneedle pores after about 48 hours. Figure 5 also shows untreated and undamaged TEWL readings which remain low and constant over the 7 day trial. Trial 2 in both subjects 1 and 2 showed more modest improvements in water loss. TEWL values showed enhanced rate of water loss for 4 days (Figure 6) and 3 days (Figure 7) for subjects 1 and 2.

Since COX-1 is apparently present at all times in tissues and COX-2 must be actively induced to exert expression, non-specific COX inhibitors would likely enhance microneedle pore viability because both COX enzymes would be sequestered and unavailable to initiate the cascade of events that result in wound healing. Both COX 1 and COX 2 inhibitors may serve to enhance the viability of microneedle pores for specifically designed patches that require different amounts of time (e.g., between about 1 day and about 7 days) for the patch to be worn by a patient.

The use of the terms "a" and "an" and "the" and similar references in the context of this disclosure (especially in the context of the following claims) are to be construed to cover both the singular and the plural, unless otherwise indicated herein or clearly contradicted by context. All methods and individual method steps described herein can be performed in any suitable order or simultaneously unless otherwise indicated herein or otherwise clearly contradicted by context. The use of any and all examples, or exemplary language (e.g., such as, preferred, preferably) provided herein, is intended merely to further illustrate the content of the disclosure and does not pose a limitation on the scope, or range of equivalents, to which the appended claims are entitled. No language in the specification should be construed as indicating any non-claimed element as essential to the practice of the present disclosure.

Alternative embodiments of the claimed disclosure are described herein, including the best mode known to the inventors for practicing the claimed invention. Of these, variations of the disclosed embodiments will become apparent to those of ordinary skill in the art upon reading the foregoing disclosure.

The use of individual numerical values is stated as approximations as though the values were preceded by the word "about" or "approximately." Similarly, the numerical values in the various ranges specified in this application, unless expressly indicated otherwise, are stated as approximations as though the minimum and maximum values within the stated ranges were both preceded by the word "about" or "approximately." In this manner, variations above and below the stated ranges can be used to achieve substantially the same results as values within the ranges. As used herein, the terms "about" and "approximately" when referring to a numerical value shall have their plain and ordinary meanings to a person of ordinary skill in the art to which the disclosed subject matter is most closely related or the art relevant to the range or element at issue. The amount of broadening from the strict numerical boundary depends upon many factors. For example, some of the factors which may be considered include the criticality of the element and/or the effect a given amount of variation will have on the performance of the claimed subject matter, as well as other considerations known to those of skill in the art. As used herein, the use of differing amounts of significant digits for different numerical values is not meant to limit how the use of the words "about" or "approximately" will serve to broaden a particular numerical value or range. Thus, as a general matter, "about" or "approximately" broaden the numerical value. Also, the disclosure of ranges is intended as a continuous range including every value between the minimum and maximum values plus the broadening of the range afforded by the use of the term "about" or "approximately." Thus, recitation of ranges of values herein are merely intended to serve as a shorthand method of referring individually to each separate value falling within the range, unless otherwise indicated herein, and each separate value is incorporated into the specification as if it were individually recited herein.

## Claims

1. A transdermal drug delivery system comprising:
a. a first array of microneedles,
b. a first COX inhibitor selected from the group consisting of: a non-specific COX inhibitor, a COX-1 inhibitor, and a COX-2 inhibitor, and
c. an active pharmaceutical agent;
wherein the first COX inhibitor is different from the active pharmaceutical agent.

2. The transdermal drug delivery system of claim 1, wherein the nonspecific COX inhibitor is selected from a group consisting of: aspirin, diclofenac, diflunisal, fenoprofen, flurbiprofen, ibuprofen, indomethacin, ketoprofen, ketorolac, mefenamic acid, meloxicam, nabumetone, naproxen, olsalazine, oxaprozin, piroxicam, salsalate, sulfasalazine, sulindac, and tolmetin;
the COX-1 inhibitor is selected from the group consisting of: salicylates, mofezolac, SC-560, and FR122047; and the COX-2 inhibitor is selected from the group consisting of: nimesulide, etodolac, celecoxib, rofecoxib, valdecoxib, parecoxib, lumiracoxib and etoricoxib.

3. The transdermal drug delivery system of claim 1, wherein the active pharmaceutical agent is selected from the group consisting of: a second COX inhibitor which is different from the first COX inhibitor, proteins, nucleotides, peptides, antibodies, vaccines, macro-molecules, nanoparticles and hydrophilic molecules.

4. A composition for transdermal administration comprising:
a. a first COX inhibitor selected from the group consisting of: a non-specific COX inhibitor, a COX-1 inhibitor, and a COX-2 inhibitor; and
b. an active pharmaceutical agent;
wherein the active pharmaceutical agent is transdermally delivered by use of microneedles and the first COX inhibitor is different from the active pharmaceutical agent.

5. The composition of claim 4, wherein the first COX inhibitor is selected from the group consisting of: a non-specific COX inhibitor selected from a group consisting of: aspirin, diclofenac, diflunisal, fenoprofen, flurbiprofen, ibuprofen, indomethacin, ketoprofen, ketorolac, mefenamic acid, meloxicam, nabumetone, naproxen, olsalazine, oxaprozin, piroxicam, salsalate, sulfasalazine, sulindac, and tolmetin; a COX-1 inhibitor is selected from the group consisting of: salicylates, mofezolac, SC-560, and FR122047; and a COX-2 inhibitor is selected from the group consisting of: nimesulide, etodolac, celecoxib, rofecoxib, valdecoxib, parecoxib, lumiracoxib and etoricoxib.

6. The composition of claim 4, wherein the active pharmaceutical agent is selected from the group consisting of: a second COX inhibitor which is different from the first COX inhibitor, proteins, nucleotides, peptides, antibodies, vaccines, macro-molecules, nanoparticles and hydrophilic molecules.

7. The use of the transdermal drug delivery system in the manufacture of a medicament for the treatment of a condition selected from the group consisting of: pain disorders, cardiovascular disorders, respiratory disorders, gastrointestinal disorders, renal disorders, psychiatric disorders, endocrinological disorders, gynecological and obstetric disorders, immunological disorders, bone and joint disorders, hematological disorders, oncologic disorders, nutritional disorders, and infectious diseases; comprising
a. contacting the skin with a first array of microneedles; and
b. applying a composition comprising a therapeutically effective quantity of:
(i) an active pharmaceutical agent to be transdermally delivered; and
(ii) a first COX inhibitor selected from the group consisting of: a non-specific COX inhibitor, a COX-1 inhibitor, and a COX-2 inhibitor;
wherein the first COX inhibitor is different from the active pharmaceutical agent.

8. The use of the transdermal drug delivery system in the manufacture of a medicament of claim 7, wherein the first COX inhibitor is selected from the group consisting of: a non-specific COX inhibitor is selected from a group consisting of: aspirin, diclofenac, diflunisal, fenoprofen, flurbiprofen, ibuprofen, indomethacin, ketoprofen, ketorolac, mefenamic acid, meloxicam, nabumetone, naproxen, olsalazine, oxaprozin, piroxicam, salsalate, sulfasalazine, sulindac, and tolmetin; a COX-1 inhibitor is selected from the group consisting of: salicylates, mofezolac, SC-560, and FR122047; and a COX-2 inhibitor is selected from the group consisting of: nimesulide, etodolac, celecoxib, rofecoxib, valdecoxib, parecoxib, lumiracoxib and etoricoxib.

9. The use of the transdermal drug delivery system in the manufacture of a medicament of claim 7, wherein the active pharmaceutical agent is selected from the group consisting of:
a second COX inhibitor which is different from the first COX inhibitor, proteins, nucleotides, peptides, antibodies, vaccines, macro-molecules, nanoparticles and
hydrophilic molecules.

10. A composition for use in prolonging microneedle pore viability comprising an active pharmaceutical agent to be transdermally delivered and a first COX inhibitor selected from the group consisting of: a non-specific COX inhibitor, a COX-1 inhibitor, and a COX-2 inhibitor; wherein the first COX inhibitor is different from the active pharmaceutical agent.

11. The composition for the use of claim 10, wherein the first COX inhibitor is selected from the group consisting of: a non-specific COX inhibitor is selected from a group consisting of: aspirin, diclofenac, diflunisal, fenoprofen, flurbiprofen, ibuprofen, indomethacin, ketoprofen, ketorolac, mefenamic acid, meloxicam, nabumetone, naproxen, olsalazine, oxaprozin, piroxicam, salsalate, sulfasalazine, sulindac, and tolmetin; a COX-1 inhibitor is selected from the group consisting of: salicylates, mofezolac, SC-560, and FR122047; and a COX-2 inhibitor is selected from the group consisting of: nimesulide, etodolac, celecoxib, rofecoxib, valdecoxib, parecoxib, lumiracoxib and etoricoxib.

12. The composition for the use of claim 10, wherein the active pharmaceutical agent is selected from the group consisting of: a second COX inhibitor which is different from the first COX inhibitor, proteins, nucleotides, peptides, antibodies, vaccines, macro-molecules, nanoparticles and hydrophilic molecules.

## Patentansprüche

1. Ein System zur transdermalen Medikamentenverabreichung umfassend:
a. eine erste Anordnung von Mikronadeln,
b. einen ersten COX-Inhibitor ausgewählt aus der Gruppe bestehend aus: einem nichtspezifischen COX-Inhibitor, einem COX-1-Inhibitor und einem COX-2-Inhibitor, und
c. ein aktives pharmazeutisches Agens;
wobei der erste COX-Inhibitor vom aktiven pharmazeutischen Agens verschieden ist.

2. Das System zur transdermalen Medikamentenverabreichung aus Anspruch 1, wobei der nichtspezifische COX-Inhibitor ausgewählt ist aus einer Gruppe bestehend aus: Aspirin, Diclofenac, Diflunisal, Fenoprofen, Flurbiprofen, Ibuprofen, Indomethacin, Ketoprofen, Ketoxolac, Mefenaminsäure, Meloxicam, Nabumeton, Naproxen, Olsalazin, Oxaprozin, Piroxicam, Salsalat, Sulfasalazin, Sulindac und Tolmetin;
der COX-1-Inhibitor ausgewählt ist aus der Gruppe bestehend aus: Salicylaten, Mofezolac, SC-560 und FR122047; und der COX-2-Inhibitor ausgewählt ist aus der Gruppe bestehend aus: Nimesulid, Etodolac, Celecoxib, Rofecoxib, Valdecoxib, Parecoxib, Lumiracoxib und Etoricoxib.

3. Das System zur transdermalen Medikamentenverabreichung aus Anspruch 1, wobei das aktive pharmazeutische Agens ausgewählt ist aus der Gruppe bestehend aus: einem zweiten COX-Inhibitor, der verschieden ist von dem ersten COX-Inhibitor, Proteinen, Nukleotiden, Peptiden, Antikörpern, Impfstoffen, Makromolekülen, Nanopartikeln und hydrophilen Molekülen.

4. Eine Zusammensatzung zur transdermalen Verabreichung umfassend:
a. einen ersten COX-Inhibitor ausgewählt aus der Gruppe bestehend aus: einem nichtspezifischen COX-Inhibitor, einem COX-1-Inhibitor und einem COX-2-Inhibitor, und
b. ein aktives pharmazeutisches Agens;
wobei das aktive pharmazeutische Agens unter Verwendung von Mikronadeln transdermal verabreicht wird und der erste COX-Inhibitor vom aktiven pharmazeutischen Agens verschieden ist.

5. Die Zusammensetzung aus Anspruch 4, wobei der erste COX-Inhibitor ausgewählt ist aus der Gruppe bestehend aus: einem nichtspezifischen COX-Inhibitor ausgewählt aus einer Gruppe bestehend aus: Aspirin, Diclofenac, Diflunisal, Fenoprofen, Flurbiprofen, Ibuprofen, Indomethacin, Ketoprofen, Ketorolac, Mefenaminsäure, Meloxicam, Nabumeton, Naproxen, Olsalazin, Oxaprozin, Piroxicam, Salsalat, Sulfasalazin, Sulindac und Tolmetin; einem COX-1-Inhibitor ausgewählt ist aus der Gruppe bestehend aus: Salicylaten, Mofezolac, SC-560 und FR122047; und einem COX-2-Inhibitor ausgewählt ist aus der Gruppe bestehend aus: Nimesulid, Etodolac, Celecoxib, Rofecoxib, Valdecoxib, Parecoxib, Lumiracoxib und Etoricoxib.

6. Die Zusammensetzung aus Anspruch 4, wobei das aktive pharmazeutische Agens ausgewählt ist aus der Gruppe bestehend aus: einem zweiten COX-Inhibitor, der verschieden ist von dem ersten COX-Inhibitor, Proteinen, Nukleotiden, Peptiden, Antikörpern, Impfstoffen, Makromolekülen, Nanopartikeln und hydrophilen Molekülen.

7. Die Verwendung des Systems zur transdermalen Medikamentenverabreichung in der Herstellung eines Medikaments zur Behandlung einer Erkrankung ausgewählt aus der Gruppe bestehend aus: Schmerzstörungen, Herz-Kreislauf-Störungen, Atemwegsstörungen, Magen-Darm-Störungen, Nierenstörungen, psychiatrischen Störungen, endokrinologischen Störungen, gynäkologischen und obstetrischen Störungen, immunologischen Störungen, Knochen- und Gelenksstörungen, hämatologischen Störungen, onkologischen Störungen, Ernährungsstörungen und Infektionskrankheiten; umfassend
a. Kontaktieren der Haut mit einer ersten Anordnung von Mikronadeln; und
b. Auftragen einer Zusammensetzung umfassend eine therapeutisch wirksame Menge von:
(i) einem aktiven pharmazeutischen Agens, das trandermal verabreicht werden soll; und
(ii) einem ersten COX-Inhibitor ausgewählt aus der Gruppe bestehend aus: einem nichtspezifischen COX-Inhibitor, einem COX-1-Inhibitor und einem COX-2-Inhibitor;
wobei der erste COX-Inhibitor vom aktiven pharmazeutischen Agens verschieden ist.

8. Die Verwendung des Systems zur transdermalen Medikamentenverabreichung in der Herstellung eines Medikaments aus Anspruch 7, wobei der erste COX-Inhibitor ausgewählt ist aus der Gruppe bestehend aus: einem nichtspezifischen COX-Inhibitor ausgewählt ist aus einer Gruppe bestehend aus: Aspirin, Diclofenac, Diflunisal, Fenoprofen, Flurbiprofen, Ibuprofen, Indomethacin, Ketoprofen, Ketorolac, Mefenaminsäure, Meloxicam, Nabumeton, Naproxen, Olsalazin, Oxaprozin, Piroxicam, Salsalat, Sulfasalazin, Sulindac und Tolmetin; einem COX-1-Inhibitor ausgewählt ist aus der Gruppe bestehend aus: Salicylaten, Mofezolac, SC-560 und FR122047; und einem COX-2-Inhibitor ausgewählt ist aus der Gruppe bestehend aus: Nimesulid, Etodolac, Celecoxib, Rofecoxib, Valdecoxib, Parecoxib, Lumiracoxib und Etoricoxib.

9. Die Verwendung des Systems zur transdermalen Medikamentenverabreichung in der Herstellung eines Medikaments aus Anspruch 7, wobei das aktive pharmazeutische Agens ausgewählt ist aus der Gruppe bestehend aus: einem zweiten COX-Inhibitor, der verschieden ist von dem ersten COX-Inhibitor, Proteinen, Nukleotiden, Peptiden, Antikörpern, Impfstoffen, Makromolekülen, Nanopartikeln und hydrophilen Molekülen.

10. Eine Zusammensetzung zur Verwendung in der Verlängerung der Lebensdauer einer Mikronadelpore umfassend ein aktives pharmazeutisches Agens, das trandermal verabreicht werden soll und einen ersten COX-Inhibitor ausgewählt aus der Gruppe bestehend aus: einem nichtspezifischen COX-Inhibitor, einem COX-1-Inhibitor und einem COX-2-Inhibitor; wobei der erste COX-Inhibitor vom aktiven pharmazeutischen Agens verschieden ist.

11. Die Zusammensetzung zur Verwendung aus Anspruch 10, wobei der erste COX-Inhibitor ausgewählt ist aus der Gruppe bestehend aus: einem nichtspezifischen COX-Inhibitor ausgewählt ist aus einer Gruppe bestehend aus: Aspirin, Diclofenac, Diflunisal, Fenoprofen, Flurbiprofen, Ibuprofen, Indomethacin, Ketoprofen, Ketorolac, Mefenaminsäure, Meloxicam, Nabumeton, Naproxen, Olsalazin, Oxaprozin, Piroxicam, Salsalat, Sulfasalazin, Sulindac und Tolmetin; einem COX-1-Inhibitor ausgewählt ist aus der Gruppe bestehend aus: Salicylaten, Mofezolac, SC-560 und FR122047; und einem COX-2-Inhibitor ausgewählt ist aus der Gruppe bestehend aus: Nimesulid, Etodolac, Celecoxib, Rofecoxib, Valdecoxib, Parecoxib, Lumiracoxib und Etoricoxib.

12. Die Zusammensetzung zur Verwendung aus Anspruch 10, wobei das aktive pharmazeutische Agens ausgewählt ist aus der Gruppe bestehend aus: einem zweiten COX-Inhibitor, der verschieden ist von dem ersten COX-Inhibitor, Proteinen, Nukleotiden, Peptiden, Antikörpern, Impfstoffen, Makromolekülen, Nanopartikeln und hydrophilen Molekülen.

## Revendications

1. Système d'administration de médicament par voie transdermique, comprenant :
a. un premier réseau de micro-aiguilles
b. un premier inhibiteur de COX choisi dans le groupe constitué par : un inhibiteur de COX non spécifique, un inhibiteur de COX-1 et un inhibiteur de COX-2, et
c. un agent pharmaceutique actif ;
dans lequel le premier inhibiteur de COX est différent de l'agent pharmaceutique actif.

2. Système d'administration de médicament par voie transdermique selon la revendication 1, dans lequel l'inhibiteur de COX non spécifique est choisi dans le groupe constitué par : l'aspirine, le diclofénac, le diflunisal, le fénoprofène, le flurbiprofène, l'ibuprofène, l'indométhacine, le kétoprofène, le kétorolac, l'acide méfénamique, le méloxicam, la nabumétone, le naproxène, l'olsalazine, l'oxaprozine, le piroxicam, le salsalate, la sulfasalazine, le sulindac et la tolmétine ;
l'inhibiteur de COX-1 est choisi dans le groupe constitué par : les salicylates, le mofézolac, le SC-560 et le FR122047 ; et l'inhibiteur de COX-2 est choisi dans le groupe constitué par : le nimésulide, l'étodolac, le célécoxib, le rofécoxib, le valdécoxib, le parécoxib, le lumiracoxib et l'étoricoxib.

3. Système d'administration de médicament par voie transdermique selon la revendication 1, dans lequel l'agent pharmaceutique actif est choisi dans le groupe constitué par : un second inhibiteur de COX qui est différent du premier inhibiteur de COX, les protéines, les nucléotides, les peptides, les anticorps, les vaccins, les macromolécules, les nanoparticules et les molécules hydrophiles.

4. Composition pour administration transdermique, comprenant :
a. un premier inhibiteur de COX choisi dans le groupe constitué par : un inhibiteur de COX non spécifique, un inhibiteur de COX-1 et un inhibiteur de COX-2, et
b. un agent pharmaceutique actif ;
dans lequel l'agent pharmaceutique actif est administré par voie transdermique au moyen de micro-aiguilles et le premier inhibiteur de COX est différent de l'agent pharmaceutique actif.

5. Composition selon la revendication 4, dans laquelle le premier inhibiteur de COX est choisi dans le groupe constitué par : un inhibiteur de COX non spécifique choisi dans un groupe constitué par : l'aspirine, le diclofénac, le diflunisal, le fénoprofène, le flurbiprofène, l'ibuprofène, l'indométhacine, le kétoprofène, le kétorolac, l'acide méfénamique, le méloxicam, la nabumétone, le naproxène, l'olsalazine, l'oxaprozine, le piroxicam, le salsalate, la sulfasalazine, le sulindac et la tolmétine ; un inhibiteur de COX-1 est choisi dans le groupe constitué par : les salicylates, le mofézolac, le SC-560 et le FR122047 ; et un inhibiteur de COX-2 est choisi dans le groupe constitué par : le nimésulide, l'étodolac, le célécoxib, le rofécoxib, le valdécoxib, le parécoxib, le lumiracoxib et l'étoricoxib.

6. Composition selon la revendication 4, dans laquelle l'agent pharmaceutique actif est choisi dans le groupe constitué par : un second inhibiteur de COX qui est différent du premier inhibiteur de COX, les protéines, les nucléotides, les peptides, les anticorps, les vaccins, les macromolécules, les nanoparticules et les molécules hydrophiles.

7. Utilisation du système d'admmistration de médicament par voie transdermique dans la fabrication d'un médicament destiné au traitement d'une affection choisie dans le groupe constitué par : les troubles liés à la douleur, les troubles cardio-vasculaires, les troubles respiratoires, les troubles gastro-intestinaux, les troubles rénaux, les troubles psychiatriques, les troubles endocrinologiques, les troubles gynécologiques et obstétriques, les troubles immunologiques, les troubles des os et des articulations, les troubles hématologiques, les troubles oncologiques, les troubles nutritionnels et les maladies infectieuses ; comprenant
a. la mise en contact de la peau avec un premier réseau de micro-aiguilles ; et
b. l'application d'une composition comprenant une quantité thérapeutiquement efficace :
(i) d'un agent pharmaceutique actif à administrer par voie transdermique ; et
(ii) d'un premier inhibiteur de COX choisi dans le groupe constitué par : un inhibiteur de COX non spécifique, un inhibiteur de COX-1 et un inhibiteur de COX-2;
dans lequel le premier inhibiteur de COX est différent de l'agent pharmaceutique actif.

8. Utilisation du système d'administration de médicament par voie transdermique dans la fabrication d'un médicament selon la revendication 7, dans laquelle le premier inhibiteur de COX est choisi dans le groupe constitué par : un inhibiteur de COX non spécifique choisi dans un groupe constitué par : l'aspirine, le diclofénac, le diflunisal, le fénoprofène, le flurbiprofène, l'ibuprofène, l'indométhacine, le kétoprofène, le kétorolac, l'acide méfénamique, le méloxicam, la nabumétone, le naproxène, l'olsalazine, l'oxaprozine, le piroxicam, le salsalate, la sulfasalazine, le sulindac et la tolmétine ; un inhibiteur de COX-1 est choisi dans le groupe constitué par : les salicylates, le mofézolac, le SC-560 et le FR122047 ; et un inhibiteur de COX-2 est choisi dans le groupe constitué par : le nimésulide, l'étodolac, le célécoxib, le rofécoxib, le valdécoxib, le parécoxib, le lumiracoxib et l'étoricoxib.

9. Utilisation du système d'administration de médicament par voie transdermique dans la fabrication d'un médicament selon la revendication 7, dans laquelle l'agent pharmaceutique actif est choisi dans le groupe constitué par : un second inhibiteur de COX qui est différent du premier inhibiteur de COX, les protéines, les nucléotides, les peptides, les anticorps, les vaccins, les macromolécules, les nanoparticules et les molécules hydrophiles.

10. Composition destiné à une utilisation pour accroître la durabilité de pores créés par des micro-aiguilles, comprenant un agent pharmaceutique actif à administrer par voie transdermique et un premier inhibiteur de COX choisi dans le groupe constitué par : un inhibiteur de COX non spécifique, un inhibiteur de COX-1 et un inhibiteur de COX-2 ; dans lequel le premier inhibiteur de COX est différent de l'agent pharmaceutique actif.

11. Composition destiné à une utilisation selon la revendication 10, dans laquelle le premier inhibiteur de COX est choisi dans le groupe constitué par : un inhibiteur de COX non spécifique choisi dans un groupe constitué par : l'aspirine, le diclofénac, le diflunisal, le fénoprofène, le flurbiprofène, l'ibuprofène, l'indométhacine, le kétoprofène, le kétorolac, l'acide méfénamique, le méloxicam, la nabumétone, le naproxène, l'olsalazine, l'oxaprozine, le piroxicam, le salsalate, la sulfasalazine, le sulindac et la tolmétine; un inhibiteur de COX-1 est choisi dans le groupe constitué par : les salicylates, le mofézolac, le SC-560 et le FR122047 ; et un inhibiteur de COX-2 est choisi dans le groupe constitué par : le nimésulide, l'étodolac, le célécoxib, le rofécoxib, le valdécoxib, le parécoxib, le lumiracoxib et l'étoricoxib.

12. Composition destiné à une utilisation selon la revendication 10, dans laquelle l'agent pharmaceutique actif est choisi dans le groupe constitué par : un second inhibiteur de COX qui est différent du premier inhibiteur de COX, les protéines, les nucléotides, les peptides, les anticorps, les vaccins, les macromolécules, les nanoparticules et les molécules hydrophiles.
